# EUROPÄISCHE PATENTSCHRIFT

(11) **EP 1 088 486 B1**
(45) Veröffentlichungstag und Bekanntmachung des Hinweises auf die Patenterteilung: **08.03.2006**
(21) Anmeldenummer: 00118467.0
(22) Anmeldetag: 25.08.2000
(51) Int. Cl.: A23L 1/303, A23P 1/06, A23L 1/0562, A61K 9/16, A23J 3/06, A61K 31/355, C07D 311/72

(54) **Stabile pulverförmige Vitamin-und/oder Carotinoid-Präparate und Verfahren zu deren Herstellung**
Stable powder compositions comprising vitamins and/or carotenoids and their preparation
Composition pulverulente stable comprenant vitamines et/ou carotenoids et leur préparation

(30) Priorität: 24.09.1999 US 404600
(43) Veröffentlichungstag der Anmeldung: 04.04.2001
(73) Patentinhaber: BASF Aktiengesellschaft, 67056 Ludwigshafen (DE)
(72) Erfinder: Bewert, Wolfgang, Dr., 67227 Frankenthal (DE); Schmitt, Peter, 67069 Ludwigshafen (DE); Betz, Roland, Dr., 67150 Niederkirchen (DE); Bower, David Kenneth, Trenton, MI 48183 (US); Chaundy, Frederick Kenneth, Grosse Ile, MI 48138 (US); Laas, Harald, Dr., 67133 Maxdorf (DE)

(56) Entgegenhaltungen:
- US-A- 4 262 017
- US-A- 4 294 856
- US-A- 5 126 328
- US-A- 5 153 177
- US-A- 5 356 636

## Beschreibung

Die Erfindung betrifft stabile pulverförmige Vitamin- und/oder Carotinoid-Präparate und Verfahren zu deren Herstellung.

Pulverförmige Vitamin- und Carotinoid-Präparate sind allgemein bekannt und werden in der pharmazeutischen Industrie sowie in der Nahrungs- und Futtermittelindustrie in großem Umfang verwendet. So sind in der Literatur viele Verfahren zur Herstellung geeigneter Präparate beschrieben.

In der Regel dispergiert man die fettlöslichen Vitamine und/oder Carotinoide in einer wäßrigen Lösung eines organischen filmbildenden Kolloids und überführt die erhaltene Dispersion letztlich in trockene pulverförmige Präparate.

Als filmbildendes Kolloid wird nach dem Stand der Technik üblicherweise Gelatine verwendet.

Besonders hohe Ansprüche werden an die Stabilität solcher Präparate gestellt, wenn diese als Zusatz zu Lebensmitteln oder zu Tierfutter verwendet werden sollen, da sie bei dieser Verwendung vielfach Einflüssen, wie erhöhten Temperaturen, Feuchtigkeit, mechanische Reibung oder Druck ausgesetzt sind, die für die empfindlichen Vitamine und Carotinoide äußerst schädlich sind. Es hat daher nicht an Versuchen gefehlt, Verfahren zu entwickeln, die thermisch und mechanisch besonders stabile Präparate liefern.

So ist beispielsweise aus GB 993 138 bekannt, Gelatine-haltige Vitaminpräparate dadurch zu stabilisieren, daß man die Partikel mit einem Gelatine-denaturierenden Mittel, wie Formaldehyd, Glyoxal, Acetaldehyd oder Dihydroxyaceton, behandelt und anschließend erhitzt oder aber nur einer Wärmebehandlung unterzieht.

Aus EP-B-0 285 682 ist ein Verfahren zur Herstellung von kugelförmigen Präparaten, welche fettlösliche Vitamine enthalten, durch Emulsionsbildung mittels Wasser, Gelatine und einem Zucker, Umwandlung der Emulsion in Tröpfchen, Sammeln der Tröpfchen in einer Masse aus Stärkepulver in einer Art, daß die Tröpfchen voneinander getrennt bleiben, bis sich ihre Form dauerhaft ausgebildet hat, Trennen der erhaltenen Partikel von überschüssigem Stärkepulver und anschließende Wärmebehandlung bei Temperaturen von 90 bis 180°C bekannt.

Ferner beschreibt EP-A-0 494 417 ein Verfahren zur Vernetzung von Gelatine in Gegenwart eines reduzierenden Zuckers und eines wasserlöslichen Salzes einer Carbonsäure oder einer anorganischen Säure bei Temperaturen im Bereich von 55 bis 180°C.

Die oben genannten Verfahren haben jedoch den Nachteil, daß die erforderlichen Vernetzungszeiten häufig zu lang bzw. die Vernetzungstemperaturen zu hoch sind, so daß es zu Produktschädigungen der thermisch instabilen fettlöslichen Vitamine bzw. Carotinoide kommen kann.

Es war daher die Aufgabe der Erfindung, ein Verfahren zur Herstellung stabiler, pulverförmiger Vitamin- und/oder Carotinoid-Präparate bereitzustellen, welches die o.g. Nachteile nicht aufweist.

Diese Aufgabe wurde gelöst durch ein Verfahren zur Herstellung von stabilen, heißwasserunlöslichen Trockenpulvern, welche ein oder mehrere fettlösliche Vitamine und/oder ein oder mehrere Carotinoide enthalten, welches folgende Verfahrensschritte beinhaltet:
A. Herstellung einer wäßrigen Dispersion, enthaltend:
   a1) 2 bis 50 Gew.-% mindestens eines Proteins,
   a2) 1 bis 30 Gew.-% mindestens eines Zuckers,
   a3) 0,2 bis 20 Gew.-% mindestens eines anorganischen Salzes,
   a4) 0,1 bis 20 Gew.-% mindestens eines fettlöslichen Vitamins und/oder mindestens eines Carotinoids,
   a5) 5 bis 95 Gew.-% Wasser,

   wobei sich alle Gew.-% Angaben auf das Gesamtgewicht der wäßrigen Dispersion beziehen und die Summe der Prozentangaben der Einzelkomponenten a1) bis a5) 100% ergibt,
B. Überführung dieser Dispersion in ein Trockenpulver und
C. Erhitzen des Trockenpulvers auf eine Temperatur im Bereich von 55°C bis 180°C,

dadurch gekennzeichnet, daß man als anorganisches Salz a3) Alkaliphosphate verwendet, so daß das Protein dabei in einem solchen Ausmaß vernetzt wird, daß das Trockenpulver nach Einbringen in Wasser von 100°C mindestens 3 Minuten lang wasserunlöslich ist.

Die als Schutzkolloid bei der Herstellung der wäßrigen Dispersion im Verfahrensschritt (A) eingesetzten Proteine a1) können sowohl pflanzlichen als auch tierischen Ursprungs sein. Als Beispiele seien insbesondere Gelatine, u.a. Knochengelatine, Rindergelatine, Fischgelatine, jeweils des A- und B-Typs in einem weiten Bloombereich sowie Pektin, Casein oder Caseinat, Sojaproteine und Maisproteine genannt. Bevorzugt verwendet man Gelatine mit einem Bloomwert von 50 bis 300, besonders bevorzugt von 80 bis 150. Das Schutzkolloid verwendet man im allgemeinen in Mengen von etwa 2 bis 50 Gew.-%, bevorzugt 3 bis 25 Gew.-%, besonders bevorzugt 5 bis 15 Gew.-%, bezogen auf das Gesamtgewicht der wäßrigen Dispersion.

Als Komponente a2) können alle reduzierenden Zucker oder Zuckersirupe mit Anteilen von reduzierenden Zuckern verwendet werden. Zu reduzierenden Zuckern zählen u.a. Fructose, Glucose, Lactose, Maltose, Xylose, Arabinose, Ribose und Saccharose sowie Honig, Fructose- und Glucose-Sirupe.

Bevorzugt verwendete Zucker im Rahmen der Erfindung sind Fructose, Glucose und Saccharose sowie deren Mischungen. Besonders bevorzugte Zucker sind Glucose und/oder Fructose. Die Zucker verwendet man im allgemeinen in Mengen von etwa 1 bis 30 Gew.-%, bevorzugt 2 bis 20 Gew.-%, besonders bevorzugt 3 bis 15 Gew.-%, bezogen auf das Gesamtgewicht der wäßrigen Dispersion.

Zu den fettlöslichen Vitaminen der Komponente a4) zählen die Vitamine A, E, D und K einschließlich deren Derivate, beispielsweise Vitamin A-Ester wie Vitamin A-Acetat, Vitamin A-Propionat oder Vitamin A-Palmitat sowie Vitamin E-Ester wie Tocopherylacetat. Sie können im Rahmen der Erfindung in Form von Vitaminlösungen in Ölen, als Provitamine sowie als reine Vitamine natürlichen oder synthetischen Ursprungs eingesetzt werden. Von besonderem Interesse sind Vitamin A und dessen Derivate, besonders bevorzugt Vitamin A-Acetat, Vitamin A-Propionat und Vitamin A-Palmitat sowie deren Mischungen, ganz besonders bevorzugt Vitamin A-Acetat.

Unter Carotinoiden werden Verbindungen verstanden wie β-Carotin, Lycopin, Bixin, Zeaxanthin, Citranaxanthin, Canthaxanthin, Astaxanthin, Lutein, Capsanthin, Cryptoxanthin, β-Apo-8'-carotinsäure und deren Ester, β-Apo-8'-carotinal, β-Apo-12'-carotinal und deren Mischungen. Bevorzugte Carotinoide sind β-Carotin, Lycopin, Lutein, Zeaxanthin, Canthaxanthin und Astaxanthin.

Die Anteile an Vitaminen und/oder Carotinoiden betragen im allgemeinen 0,1 bis 20 Gew.-%, vorzugsweise 1 bis 15 Gew.-%, besonders bevorzugt 2 bis 12 Gew.-%, bezogen auf die Gesamtmasse der wäßrigen Dispersion, erhältlich nach Verfahrensschritt (A).

Erfindungsgemäß wird als anorganisches Salz a3) mindestens ein Alkaliphosphat zur Herstellung der o.g. wäßrigen Dispersion eingesetzt. Dabei kann es sich beispielsweise um Natrium-, Kalium-oder Lithiumsalze sowohl der Mono-, Di- und Triphosphorsäure als auch von Polyphosphorsäure handeln.

Bevorzugte Alkaliphosphate sind tertiäres Natriumphosphat, Natriumdihydrogenphosphat, Dinatriumhydrogenphosphat, Dinatriumdihydrogendiphosphat, Pentanatriumtriphosphat, Natriumtrimethaphosphat, tertiäres Kaliumphosphat, Kaliumdihydrogenphosphat, Dikaliumhydrogenphosphat, Dikaliumhydrogendiphosphat, Pentakaliumtriphosphat, Kaliumtrimethaphosphat. Besonders bevorzugt sind tertiäres Natriumphosphat, Natriumdihydrogenphosphat, Dinatriumhydrogenphosphat, tertiäres Kaliumphosphat, Kaliumdihydrogenphosphat, Dikaliumhydrogenphosphat. Ganz besonders bevorzugt ist Dinatriumhydrogenphosphat.

Die Anteile an Alkaliphosphat betragen im allgemeinen 0,2 bis 20 Gew.-%, vorzugsweise 0,3 bis 15 Gew.-%, besonders bevorzugt 0,4 bis 10 Gew.-%, ganz besonders bevorzugt 0,5 bis 5 Gew.%, bezogen auf die Gesamtmasse der wäßrigen Dispersion, erhältlich nach Verfahrensschritt (A).

Zusätzlich zu den o. g. Bestandteilen können der Dispersion vorteilhafterweise noch andere, für die Herstellung von Wirkstofftrockenpulvern übliche Hilfs- und Zusatzstoffe, zugefügt werden.

Von besonderer Bedeutung für einen Einsatz der Trockenpulver als Futtermittelzusatz ist bei oxidationsempfindlichen Wirkstoffen ein Zusatz von Antioxidantien, wie Ethoxyquin, butyliertes Hydroxytoluol (BHT), butyliertes Hydroxyanisol (BHA) oder gegebenenfalls Tocopherol sowie Stabilisatoren, wie Zitronensäure oder Phytinsäure und deren Alkali- oder Erdalkalisalze, oder aber Komplexbildner, wie Ethylendiamintetraessigsäure (EDTA) oder Nitrilotriessigsäure (NTA).

Häufig werden der Emulsion aber auch Feuchthaltemittel, wie Glycerin, Sorbitol oder Polyethylenglykole oder auch zusätzliche Emulgatoren, wie Lecithin, zugefügt.

Darüberhinaus haben sich Zusätze, wie Stärke, insbesondere Maisstärke oder Maltodextrin oder Dickungsmittel, wie Gummiarabicum, Guargummi, Alginate und bestimmte abgebaute Stärken zur Einstellung der Viskosität der Emulsion als nützlich erwiesen.

Als vorteilhaft hat sich herausgestellt, daß man der wäßrigen Dispersion im Verfahrensschritt (A) als zusätzliche Komponente a6) 0,5 bis 20 Gew.-%, bevorzugt 1 bis 10 Gew.-% Stärke, insbesondere Maisstärke zusetzt.

Bezüglich näherer Details über Bedeutung, Art und Menge solcher Zusätze sei auf entsprechende Fachliteratur, beispielsweise auf die obengenannte Monographie "Fat-soluble Vitamins", Vol. 9, insbesondere Seiten 128 bis 133 verwiesen.

Ein besondere Ausführungsform der erfindungsgemäßen Verfahrens ist dadurch gekennzeichnet, daß man im Schritt (A)
a1) 5 bis 15 Gew.-% einer Gelatine mit einem Bloom-Wert von 50 bis 300,
a2) 3 bis 15 Gew.-% mindestens eines Zuckers, ausgewählt aus der Gruppe, bestehend aus Fructose und Glucose,
a3) 0,5 bis 5 Gew.-% Na₂HPO₄,
a4) 2 bis 12 Gew.-% mindestens eines fettlöslichen Vitamins, ausgewählt aus der Gruppe, bestehend aus Vitamin A, Vitamin A-Acetat, Vitamin E und Vitamin E-Acetat,
a5) 30 bis 85 Gew.-% Wasser und
a6) 1 bis 10 Gew.-% Stärke einsetzt.

Zur Durchführung des erfindungsgemäßen Verfahrens geht man im allgemeinen so vor, daß man zur Herstellung der Dispersion im Verfahrensschritt (A) mindestens ein Proteine a1) in heißem Wasser bei einer Temperatur von 50 bis 70°C zur Lösung bringt, zu dieser Lösung jeweils mindestens ein Zucker a2), ein Alkaliphosphat a3), ein fettlösliches Vitamin und/oder Carotinoid, Stabilisatoren und die anderen üblichen Zusatzstoffe sowie ggf. noch zusätzlich Wasser addiert und das Gemisch durch kräftiges Rühren bei erhöhter Temperatur dispergiert. Für die im letzten verarbeitungsschritt (C) erfolgende thermische Vernetzung des Pulvers sollte die fertige Dispersion in einem pH-Bereich von 4 bis 10, bevorzugt 5 bis 8 vorliegen, welcher gegebenenfalls durch Zugabe von Basen, wie NaOH, KOH, Ca(OH)₂, MgO, Soda oder NH₄OH eingestellt werden kann.

Die anschließende Weiterverarbeitung der Dispersion zu den erfindungsgemäßen Pulvern kann nach literaturbekannten Verfahren erfolgen.

Wegen der gewünschten Kornverteilung der Pulver (0,1 bis 0,6 mm Durchmesser) werden solche Verfahren bevorzugt, bei denen Vorkehrungen getroffen werden, daß die gelierten Tröpfchen der Dispersion voneinander getrennt bleiben, bis sich ihre Form stabilisiert hat.

Genannt seien beispielsweise das Verfahren gemäß EP-B-74 050, bei dem die Dispersion in hydrophobe Kieselsäure oder ein Metallsalz einer höheren Fettsäure versprüht wird oder aber das Verfahren gemäß EP-B-285 682, bei dem die Dispersion in Stärkepulver versprüht wird. Es hat sich erwiesen, daß die Versprühung mit hydrophober Kieselsäure als Puderungsmittel besonders vorteilhaft durchführbar ist.

Die nach den beschriebenen Verfahren erzeugten Pulver besitzen nach dem Trocknen (Verfahrensschritt B) einen Wassergehalt im Bereich von 5 bis 15 Gew.-%, bevorzugt im Bereich von 5 bis 10 Gew.-%. Die so erhaltenen pulverförmigen Präparate bestehen aus Teilchen mit gut ausgebildeter Oberfläche. Sie lösen sich in warmem Wasser von ca. 40°C rasch zu einer milchigen Dispersion.

Die thermische Härtung der getrockneten Pulver erfolgt im Verfahrensschritt (C) durch Erhitzen auf Temperaturen von 55 bis 180°C, wobei der dabei erfolgende Vernetzungsprozeß mit steigender Temperatur immer schneller abläuft. Bevorzugt wird die Vernetzung bei Temperaturen von 70 bis 130°C, besonders bevorzugt von 85 bis 125°C in einer Reaktionszeit von 5 Minuten bis 3 Stunden, bevorzugt 6 bis 25 Minuten durchgeführt.

Die so hergestellten Pulver weisen einen Wassergehalt im Bereich von 0,1 bis 4 Gew.-%, bevorzugt 0,5 bis 3,5 Gew.-%, besonders bevorzugt im Bereich von 1 bis 3 Gew.-% auf, sind nach Einbringen in kochendem Wasser mindestens 3 Minuten wasserunlöslich und weisen eine ausgezeichnete Stabilität bei der Lagerung (siehe dazu Beispiel 2 und Tabelle 2) sowie in praktischen Anwendungen wie Extrusion und Pelletierung auf.

Gegenüber den aus dem Stand der Technik bekannten Vernetzungsverfahren liegt der Vorteil der erfindungsgemäß verwendeten Alkaliphosphate als Vernetzungshilfsmittel in kürzeren Vernetzungszeiten (siehe dazu Tabelle 1) und damit in einer Produkt-schonenderen Fahrweise. Im Vergleich zu der in EP-A-0 494 417 beschriebenen Verwendung von Natriumacetat, mit dem sich ähnliche Vernetzungszeiten erzielen lassen, sind die erfindungsgemäß hergestellten Trockenpulver wesentlich lagerstabiler. Unerwünschte Geruchsbelästigungen sowohl bei der Vernetzungsreaktion als auch im Verlauf der Lagerung - beispielsweise durch Freisetzung von Essigsäure im Falle von Natriumacetat - können so vermieden werden.

Gegenstand der Erfindung sind auch stabile heißwasserunlösliche Trockenpulver gemäß dem eingangs beschriebenen Verfahren, enthaltend:
a1) 10 bis 70 Gew.-% mindestens eines Proteins,
a2) 5 bis 30 Gew.-% mindestens eines Zuckers,
a3) 0,5 bis 25 Gew.-% mindestens eines Alkaliphosphates,
a4) 0,1 bis 60 Gew.-% mindestens eines fettlöslichen Vitamins und/oder mindestens eines Carotinoids,
a5) 0,1 bis 4 Gew.-% Wasser,

wobei sich alle Gew.-% Angaben auf das Gesamtgewicht des Trockenpulvers beziehen und die Summe der Prozentangaben der Einzelkomponenten a1) bis a5) 100 % ergibt.

Hinsichtlich einer genaueren Definition der einzelnen Komponenten a1) bis a5) - sowohl in der allgemeinen als auch in der bevorzugten Ausführungsform - sei auf die bereits eingangs erfolgten Erläuterungen hingewiesen.

Neben den Bestandteilen a1) bis a5) kann das erfindungsgemäße Trockenpulver als Komponente a6) zusätzlich 0,5 bis 40 Gew.-% Stärke enthalten.

Bevorzugt zu nennen ist im Rahmen der Erfindung ein Trockenpulver, enthaltend
a1) 10 bis 70 Gew.-% einer Gelatine mit einem Bloom-Wert von 50 bis 300, die in einem solchen Ausmaß vernetzt ist, daß sie nach Einbringen in Wasser von 100°C mindestens 3 Minuten lang wasserunlöslich ist,
a2) 5 bis 30 Gew.-% mindestens eines Zuckers, ausgewählt aus der Gruppe, bestehend aus Fructose und Glucose,
a3) 0,5 bis 10 Gew.-% Na₂HPO₄,
a4) 1 bis 50 Gew.-% einer Verbindung, ausgewählt aus der Gruppe, bestehend aus Vitamin A, Vitamin A-Ester, Vitamin E und Vitamin E-Ester,
a5) 1 bis 3 Gew.-% Wasser.

Die Erfindung betrifft ferner Lebensmittel oder Futtermittel, die die oben genannten stabilen, heißwasserunlöslichen Trockenpulver enthalten. Unter Futtermittel sind dabei alle Arten von Vitaminvormischungen, Prämixen, Mineral- und Mischfuttermittel gemeint.

Anhand der folgenden Beispiele soll der Gegenstand der vorliegenden Erfindung näher erläutert werden.

### Beispiel 1

### Herstellung von Vitamin A Trockenpulver

Zu 300 g Wasser wurden 34,1 g (30 g Feststoff) Gelatine A 100 Bloom gegeben und nach 30-minütigem Quellen durch Erhitzen auf 60°C in Lösung gebracht. Nach Zugabe von 21,1 g Fructosesirup (15 g Feststoff, Zuckergehalt 70 %, davon 95 % Fructose in der Trockenmasse), wurden nacheinander 22,7 g Maisstärke (20 g Feststoff), 3 g Na₂HPO₄ sowie 25 g Vitamin-A-Acetat (2,19 Millionen IE/g, hergestellt aus Vitamin-A-Acetat 2,9 Millionen IE/g und stabilisiert mit 100 mg Ethoxyquin und 14,5 mg BHT pro Million IE Vitamin A) hinzugegeben. Das Gemisch wurde durch kräftiges Rühren bei 60°C emulgiert. Die hergestellte Emulsion wurde bei einer Temperatur von 55°C mit einer Einstoffdüse bei 5,5 bis 6,5 bar in einem Sprühturm in eine Wolke hydrophober Kieselsäure gesprüht. Das noch feuchte Produkt wurde auf einem Wirbeltroclaner auf eine Restfeuchte von 5 bis 6 % getrocknet und von der überschüssigen Kieselsäure abgetrennt. Anschließend wurden 10 g des erhaltenen Pulvers in einem rotierenden Aluminiumkolben, der in ein auf 110°C erhitztes Ölheizbad getaucht war, getempert. Der Vernetzungspunkt lag unter diesen Bedingungen bei 10 Minuten. Das erhaltene braune Pulver besaß einen Vitamin-A-Gehalt von 540 000 IE/g bei einem Restfeuchtegehalt von 2,9 Gew.-%.

10 g des noch nicht vernetzten Pulvers wurden alternativ bei 120°C getempert. Das erhaltene braune Pulver war unter diesen Bedingungen bereits nach 7 Minuten (= Vernetzungspunkt) in kochendem Wasser nicht mehr dispergierbar (Partikel blieben vollständig erhalten).

### Vergleichsbeispiele

Auf die gleiche Art und Weise wie in Beispiel 1 beschrieben, wurden jeweils Emulsionen mit der in der Tabelle 1 angegebenen Zusammensetzung hergestellt, zu einem Pulver versprüht und getrocknet. Beim anschließenden Tempern bei 110°C bzw. 85°C wurden die Mindestvernetzungszeiten bestimmt.

**Tabelle 1**

| Rezeptur der Emulsion | Salz-zusatz | Wassergehalt [Gew.-%] | Mindestvernetzungszeit bei 110°C [min] | Mindestvernetzungszeit bei 85°C [min] |
|---|---|---|---|---|
| | | | | |
| Vergleich: | | | | |
| | | | | |
| a) wie Bsp. 1 | - | 3,1 | > 30 | n.b. |
| b) wie Bsp. 1 | Na-Acetat | 2,9 | 12 | 190 |
| c) wie Bsp. 1 | CaHPO₄ | 2,1 | 22 | 250 |
| d) wie Bsp. 1 | Ca (H₂PO₄)₂ | 2,3 | 30 | 330 |
| e) wie Bsp. 1 | CaSO₄ | 2,0 | 27 | 260 |
| f) wie Bsp. 1 | Ca-Acetat | 2,4 | 11 | 115 |
| | | | | |
| Erfindung : | | | | |
| | | | | |
| Beispiel 1 | Na₂HPO₄ | 2,9 | 10 | 90 |
| wie Bsp. 1 | K₂HPO₄ | 2,8 | 12 | 100 |

### Beispiel 2

Aus den erhaltenen Trockenpulvern, jeweils hergestellt nach Beispiel 1 bzw. nach Vergleichsbeispiel a) wurde die Fraktion mit der Teilchengröße 250 bis 355 µm herausgesiebt und einer Stabilitätsprüfung in einen Standardprämix unterzogen. Hierzu wurden ca. 100 mg der Prüfmuster in Präperategläschen eingewogen (je Muster und Prüfzeitpunkt 4 Einwägungen), mit 4 g Prämixmischung, bestehend aus 60 % Weizengrießkleie, 30 % 50 %igem Cholinchlorid auf Kieselsäure und 10 % Spurenelementmischung, bestehend aus 37,43 % CuSO₄ × 5 H₂O; 46,78 % FeSO₄ × 7 H₂O; 11,79 % ZnO; 3,61 % MnO und 0,39 % CoCO₃ versetzt und anschließend sorgfältig mit der Hand gemischt.

Die Prüfmuster wurden in einem Klimaschrank bei konstanter Temperatur und Feuchte (40°C und 70 % rel. Feuchte) 6 Wochen lang offen gelagert. Zu Beginn der Lagerung und nach 6 Wochen wurden die 4 für den jeweiligen Prüfzeitpunkt vorbereiteten Prüfmuster entnommen und auf den verbleibenden Restgehalt an Vitamin A Wirkstoff überprüft.

Die Prüfungsergebnisse sind in Tabelle 2 dargestellt:

**Tabelle 2 Untersuchung der Lagerstabilität von Vitamin A-Trockenpulvern**

| | | |
|---|---|---|
| Trockenpulver | t=0 [I.E./g]^{*)} | t = 6 Wolchen ^{**)} |
| 1. gemaß Beispel 1 | 540.000 = 100 % | 80,5 % |
| 2. gemaß Vergleichsbeispiel a) | 520.900 = 100 % | 60,4 % |

| | | |
|---|---|---|
| *) Konzentration an Vitamin A im Trockenpulver zu Beginn der Stabilitätsuntersuchung. | | |
| **) Konzentration an Vitamin A im Trockenpulver nach 6-wöchiger Lagerung. Die Gehaltsbestimmung erfolgte UV-spektroskopisch. Der Wert gibt den Gehalt an Vitamin A, bezogen auf die Anfangskonzentration an. | | |

## Patentansprüche

1. Verfahren zur Herstellung von stabilen, heißwasserunlöslichen Trockenpulvern, welche ein oder mehrere fettlösliche Vitamine und/oder ein oder mehrere Carotinoide enthalten, welches folgende Verfahrensschritte beinhaltet:
A. Herstellung einer wäßrigen Dispersion, enthaltend:
a1) 2 bis 50 Gew.-% mindestens eines Proteins,
a2) 1 bis 30 Gew.-% mindestens eines Zuckers,
a3) 0,2 bis 20 Gew.-% mindestens eines anorganischen Salzes,
a4) 0,1 bis 20 Gew.-% mindestens eines fettlöslichen Vitamins und/oder mindestens eines Carotinoids,
a5) 5 bis 95 Gew.-% Wasser,
wobei sich alle Gew.-% Angaben auf das Gesamtgewicht der wäßrigen Dispersion beziehen und die Summe der Prozentangaben der Einzelkomponenten a1) bis a5) 100% ergibt,
B. Überführung dieser Dispersion in ein Trockenpulver und
C. Erhitzen des Trockenpulvers auf eine Temperatur im Bereich von 55°C bis 180°C,
**dadurch gekennzeichnet, daß** man als anorganisches Salz a3) Alkaliphosphate verwendet, so daß das Protein dabei in einem solchen Ausmaß vernetzt wird, daß das Trockenpulver nach Einbringen in Wasser von 100°C mindestens 3 Minuten lang wasserunlöslich ist.

2. Verfahren nach Anspruch 1, **dadurch gekennzeichnet, daß** es sich bei dem Protein a1) um mindestens eine Verbindung aus der Gruppe, bestehend aus Gelatine, Pektin, Casein und Caseinat handelt.

3. Verfahren nach einem der Ansprüche 1 oder 2, **dadurch gekennzeichnet, daß** man als Komponente a2) mindestens einen Zucker, ausgewählt aus der Gruppe, bestehend aus Fructose, Glucose und Saccharose einsetzt.

4. Verfahren nach einem der Ansprüche 1 bis 3, **dadurch gekennzeichnet, daß** man als Komponente a4) mindestens eine Verbindung aus der Gruppe, bestehend aus Vitamin A, Vitamin A-Ester, Vitamin E und Vitamin E-Ester einsetzt.

5. Verfahren nach einem der Ansprüche 1 bis 4, **dadurch gekennzeichnet, daß** die wäßrige Dispersion im verfahrensschritt A als zusätzliche Komponente a6) 0,5 bis 20 Gew.-% Stärke enthält.

6. Verfahren nach einem der Ansprüche 1 bis 5, **dadurch gekennzeichnet, daß** man im Schritt A
a1) 5 bis 15 Gew.-% einer Gelatine mit einem Bloom-Wert von 50 bis 300,
a2) 3 bis 15 Gew.-% mindestens eines Zuckers, ausgewählt aus der Gruppe, bestehend aus Fructose und Glucose,
a3) 0,5 bis 5 Gew.-% Na₂HPO₄,
a4) 2 bis 12 Gew.-% mindestens eines fettlöslichen Vitamins, ausgewählt aus der Gruppe, bestehend aus Vitamin A, Vitamin A-Acetat, Vitamin E und Vitamin E-Acetat,
a5) 30 bis 85 Gew.-% Wasser und
a6) 1 bis 10 Gew.-% Stärke einsetzt.

7. Verfahren nach einem der Ansprüche 1 bis 6, **dadurch gekennzeichnet, daß** das Trockenpulver nach Verfahrensschritt B) einen Wassergehalt im Bereich von 5 bis 15 Gew.-% aufweist.

8. Verfahren nach einem der Ansprüche 1 bis 7, **dadurch gekennzeichnet, daß** das Trockenpulver nach verfahrensschritt C) einen Wassergehalt im Bereich von 0,1 bis 4 Gew.-% aufweist.

9. Stabile heißwasserunlösliche Trockenpulver gemäß einem Verfahren nach Anspruch 1, enthaltend:
a1) 10 bis 70 Gew.-% mindestens eines Proteins,
a2) 5 bis 30 Gew.-% mindestens eines Zuckers,
a3) 0,5 bis 25 Gew.-% mindestens eines Alkaliphosphates,
a4) 0,1 bis 60 Gew.-% mindestens eines fettlöslichen Vitamins und/oder mindestens eines Carotinoids,
a5) 0,1 bis 4 Gew.-% Wasser,
wobei sich alle Gew.-% Angaben auf das Gesamtgewicht des Trockenpulvers beziehen und die Summe der Prozentangaben der Einzelkomponenten a1) bis a5) 100% ergibt.

10. Trockenpulver nach Anspruch 9, enthaltend als Protein mindestens eine Verbindung aus der Gruppe, bestehend aus Gelatine, Pektin, Casein und Caseinat.

11. Trockenpulver nach einem der Ansprüche 9 oder 10, enthaltend als Komponente a2) mindestens einen Zucker, ausgewählt aus der Gruppe, bestehend aus Fructose, Glucose und Saccharose.

12. Trockenpulver nach einem der Ansprüche 9 bis 11, enthaltend als Komponente a6) zusätzlich 0,5 bis 40 Gew.-% Stärke.

13. Trockenpulver nach einem der Ansprüche 9 bis 12, enthaltend
a1) 10 bis 70 Gew.-% einer Gelatine mit einem Bloom-Wert von 50 bis 300, die in einem solchen Ausmaß vernetzt ist, daß sie nach Einbringen in Wasser von 100°C mindestens 3 Minuten lang wasserunlöslich ist,
a2) 5 bis 30 Gew.-% mindestens eines Zuckers, ausgewählt aus der Gruppe, bestehend aus Fructose und Glucose,
a3) 0,5 bis 10 Gew.-% Na₂HPO₄,
a4) 1 bis 50 Gew.-% einer Verbindung, ausgewählt aus der Gruppe, bestehend aus Vitamin A, Vitamin A-Ester, Vitamin E und Vitamin E-Ester,
a5) 1 bis 3 Gew.-% Wasser.

14. Lebensmittel oder Futtermittel, enthaltend Trockenpulver gemäß einem der Ansprüche 9 bis 13.

## Claims

1. A process for producing a stable dry powder which is insoluble in hot water and which comprises one or more lipid-soluble vitamins and/or one or more carotenoids, which comprises the following process steps:
A. preparing an aqueous dispersion comprising:
a1) 2 to 50% by weight of at least one protein,
a2) 1 to 30% by weight of at least one sugar,
a3) 0.2 to 20% by weight of at least one inorganic salt,
a4) 0.1 to 20% by weight of at least one lipid-soluble vitamin and/or at least one carotenoid,
a5) 5 to 95% by weight of water,
where all the % by weight data are based on the total weight of the aqueous dispersion, and the total of the percentage data for the individual components a1) to a5) is 100%,
B. converting this dispersion into a dry powder and
C. heating the dry powder to a temperature in the range from 55°C to 180°C,
wherein alkali metal phosphates are used as inorganic salt a3), so that the protein is crosslinked to an extent such that the dry powder is insoluble in water for at least 3 minutes after introduction into water at 100°C.

2. The process according to claim 1, wherein the protein a1) is at least one compound from the group consisting of gelatin, pectin, casein and caseinate.

3. The process according to either of claims 1 or 2, wherein at least one sugar selected from the group consisting of fructose, glucose and sucrose is employed as component a2).

4. The process according to any of claims 1 to 3, wherein at least one compound from the group consisting of vitamin A, vitamin A ester, vitamin E and vitamin E ester is employed as component a4).

5. The process according to any of claims 1 to 4, wherein the aqueous dispersion in process step A comprises as additional component a6) from 0.5 to 20% by weight of starch.

6. The process according to any of claims 1 to 5, wherein in process step A
a1) 5 to 15% by weight of a gelatin with a Bloom value of from 50 to 300,
a2) 3 to 15% by weight of at least one sugar selected from the group consisting of fructose and glucose,
a3) 0.5 to 5% by weight of Na₂HPO₄,
a4) 2 to 12% by weight of at least one lipid-soluble vitamin selected from the group consisting of vitamin A, vitamin A acetate, vitamin E and vitamin E acetate,
a5) 30 to 85% by weight of water and
a6) 1 to 10% by weight of starch are employed.

7. The process according to any of claims 1 to 6, wherein the dry powder according to process step B) has a water content in the range from 5 to 15% by weight.

8. The process according to any of claims 1 to 7, wherein the dry powder according to process step C) has a water content in the range from 0.1 to 4% by weight.

9. A stable dry powder which is insoluble in hot water according to the process according to claim 1, comprising:
a1) 10 to 70% by weight of at least one protein,
a2) 5 to 30% by weight of at least one sugar,
a3) 0.5 to 25% by weight of at least one alkali metal phosphate,
a4) 0.1 to 60% by weight of at least one lipid-soluble vitamin and/or at least one carotenoid,
a5) 0.1 to 4% by weight of water,
where all the % by weight data are based on the total weight of the dry powder, and the total of the percentage data for the individual components a1 to a5) is 100%.

10. The dry powder according to claim 9, comprising as protein at least one compound from the group consisting of gelatin, pectin, casein and caseinate.

11. The dry powder according to either of claims 9 or 10, comprising as component a2) at least one sugar selected from the group consisting of fructose, glucose and sucrose.

12. The dry powder according to any of claims 9 to 11, comprising as component a6) additionally from 0.5 to 40% by weight of starch.

13. The dry powder according to any of claims 9 to 12, comprising
a1 10 to 70% by weight of a gelatin with a Bloom value of from 50 to 300, which is crosslinked to an extent such that it is insoluble in water for at least 3 minutes after introduction into water at 100°C,
a2) 5 to 30% by weight of at least one sugar selected from the group consisting of fructose and glucose,
a3) 0.5 to 10% by weight of Na₂HPO₄,
a4) 1 to 50% by weight of a compound selected from the group consisting of vitamin A, vitamin A ester, vitamin E and vitamin E ester and
a5) 1 to 3% by weight of water.

14. The human food or animal feed comprising the dry powder according to any of claims 9 to 13.

## Revendications

1. Procédé de préparation de poudres sèches stables, insolubles dans l'eau chaude, qui contiennent une ou plusieurs vitamines liposolubles et/ou un ou plusieurs caroténoïdes, qui comporte les étapes suivantes :
A. une préparation d'une dispersion aqueuse, contenant :
a1) 2 à 50 % en poids d'au moins une protéine,
a2) 1 à 30 % en poids d'au moins un sucre,
a3) 0,2 à 20 % en poids d'au moins un sel inorganique,
a4) 0,1 à 20 % en poids d'au moins une vitamine liposoluble et/ou d'au moins un caroténoïde,
a5) 5 à 95 % en poids d'eau,
toutes les indications en % en poids se rapportant au poids total de la dispersion aqueuse et la somme des indications en % des composants individuels a1) à a5) donnant 100 %,
B. une transformation de cette dispersion en une poudre sèche, et
C. un chauffage de la poudre sèche à une température de l'ordre de 55°C à 180°C,
**caractérisé en ce que**, comme sel inorganique a3), on utilise des phosphates de métal alcalin de façon que la protéine soit réticulée dans une mesure telle que la poudre sèche soit, après introduction dans de l'eau à 100°C, insoluble dans l'eau pendant au moins 3 minutes.

2. Procédé suivant la revendication 1, **caractérisé en ce que**, en ce qui concerne la protéine a1) il s'agit d'au moins un composé du groupe constitué de gélatine, de pectine, de caséine et de caséinate.

3. Procédé suivant l'une des revendications 1 et 2, **caractérisé en ce que**, comme composant a2), on met en oeuvre au moins un sucre choisi parmi le groupe constitué de fructose, de glucose et de saccharose.

4. Procédé suivant l'une des revendications 1 à 3, **caractérisé en ce que**, comme composant a4), on met en oeuvre au moins un composé du groupe constitué de vitamine A, d'ester de vitamine A, de vitamine E et d'ester de vitamine E.

5. Procédé suivant l'une des revendications 1 à 4, **caractérisé en ce que** la dispersion aqueuse dans l'étape A contient 0,5 à 20 % en poids d'amidon comme composant supplémentaire a6).

6. Procédé suivant l'une des revendications 1 à 5, **caractérisé en ce que**, dans l'étape A, on met en oeuvre
a1) 5 à 15 % en poids d'une gélatine présentant une valeur bloom de 50 à 300,
a2) 3 à 15 % en poids d'au moins un sucre, choisi parmi le groupe constitué de fructose et de glucose,
a3) 0,5 à 5% en poids de Na₂HPO₄ ,
a4) 2 à 12 % en poids d'au moins une vitamine liposoluble, choisie parmi le groupe constitué de vitamine A, d'acétate de vitamine A, de vitamine E et d'acétate de vitamine E,
a5) 30 à 85 % en poids d'eau, et
a6) 1 à 10% en poids d'amidon.

7. Procédé suivant l'une des revendications 1 à 6,
**caractérisé en ce que** la poudre sèche présente, après l'étape B), une teneur en eau de l'ordre de 5 à 15 % en poids.

8. Procédé suivant l'une des revendications 1 à 7, **caractérisé en ce que** la poudre sèche présente, après l'étape C), une teneur en eau de l'ordre de 0,1 à 4 % en poids.

9. Poudre sèche stable, insoluble dans l'eau chaude, suivant un procédé selon la revendication 1, contenant :
a1) 10 à 70 % en poids d'au moins une protéine,
a2) 5 à 30% en poids d'au moins un sucre,
a3) 0,5 à 25 % en poids d'au moins un phosphate de métal alcalin,
a4) 0,1 à 60 % en poids d'au moins une vitamine liposoluble et/ou d'au moins un caroténoïde,
a5) 0,1 à 4 % en poids d'eau,
toutes les indications en % en poids se rapportant au poids total de la poudre sèche et la somme des indications en % des composants individuels a1) à a5) donnant 100 %.

10. Poudre sèche suivant la revendication 9, contenant, comme protéine, au moins un composé du groupe constitué de gélatine, de pectine, de caséine et de caséinate.

11. Poudre sèche suivant l'une des revendications 9 et 10, contenant, comme composant a2), au moins un sucre choisi parmi le groupe constitué de fructose, de glucose et de saccharose.

12. Poudre sèche suivant l'une des revendications 9 à 11, contenant en supplément 0,5 à 40 % en poids d'amidon, comme composant a6).

13. poudre sèche suivant l'une des revendications 9 à 12, contenant
a1) 10 à 70 % en poids d'une gélatine présentant une valeur bloom de 50 à 300, qui est réticulée dans une mesure telle que, après introduction dans de l'eau à 100°C, elle est insoluble dans l'eau pendant au moins 3 minutes,
a2) 5 à 30% en poids d'au moins un sucre, choisi parmi le groupe constitué de fructose et de glucose,
a3) 0,5 à 10 % en poids de Na₂HPO₄,
a4) 1 à 50 % en poids d'un composé choisi parmi le groupe constitué de vitamine A, d'ester de vitamine A, de vitamine E et d'ester de vitamine E,
a5) 1 à 3% en poids d'eau.

14. Aliments ou fourrages, contenant de la poudre sèche suivant l'une des revendications 9 à 13.
